(19) Europäisches Patentamt

European Patent Office

Office européen des brevets

(11) **EP 1 323 379 B1**

(12) **EUROPÄISCHE PATENTSCHRIFT**

(45) Veröffentlichungstag und Bekanntmachung des Hinweises auf die Patenterteilung:
**20.04.2005 Patentblatt 2005/16**

(51) Int Cl.⁷: **A61B 8/00**, G01T 1/161

(21) Anmeldenummer: **02102801.4**

(22) Anmeldetag: **18.12.2002**

(54) **Verfahren zur Verbesserung der Auflösung einer nuklearmedizinischen Aufnahme**

Method for improving the resolution of a nuclear medicine image

Procédé pour améliorer la résolution d'une image de médecine nucléaire

(84) Benannte Vertragsstaaten:
**AT BE BG CH CY CZ DE DK EE ES FI FR GB GR IE IT LI LU MC NL PT SE SI SK TR**

(30) Priorität: **19.12.2001 DE 10162273**

(43) Veröffentlichungstag der Anmeldung:
**02.07.2003 Patentblatt 2003/27**

(73) Patentinhaber:
• **Philips Intellectual Property & Standards GmbH 20099 Hamburg (DE)**
Benannte Vertragsstaaten:
**DE**
• **Koninklijke Philips Electronics N.V. 5621 BA Eindhoven (NL)**
Benannte Vertragsstaaten:
**AT BE BG CY CZ ES FI EE GB GR IE IT LU MC NL PT SE TR SI SK CH DK FR LI**

(72) Erfinder: **Eck, Kai**
**Postfach 50 04 42, 52088 Aachen (DE)**

(74) Vertreter: **Volmer, Georg, Dipl.-Ing. et al**
**Philips Intellectual Property & Standards GmbH, Postfach 50 04 42**
**52088 Aachen (DE)**

(56) Entgegenhaltungen:
**US-A- 5 871 013**

• **MAINTZ J B A ET AL: "A SURVEY OF MEDICAL IMAGE REGISTRATION" MEDICAL IMAGE ANALYSIS, OXFORDUNIVERSITY PRESS, OXFORD, GB, Bd. 2, Nr. 1, 1998, Seiten 1-37, XP001032679 ISSN: 1361-8423**
• **P.A. VAN DEN ELSEN ET AL.: "Grey value correlation techniques used for automatic matching " PROC. VISUALIZATION IN BIOMEDICAL COMPUTING, Bd. 2359, Oktober 1994 (1994-10), Seiten 227-237, XP001147475**

## Beschreibung

Verfahren zur Verbesserung der Auflösung einer nuklearmedizinischen Aufnahme Die Erfindung betrifft ein Verfahren zur Verbesserung der Auflösung einer nuklearmedizinischen Aufnahme durch ihre Kombination mit einer Ultraschallaufnahme aus demselben Körpervolumen. Ferner betrifft sie eine Vorrichtung enthaltend einen Speicher und eine Datenverarbeitungseinheit, mit welcher das genannte Verfahren durchgeführt werden kann.

In der medizinischen Diagnostik haben nuklearmedizinische Verfahren einen wichtigen Platz eingenommen, da sie über die Beobachtung der Aufnahme von radioaktiven Markerstoffen die Visualisierung von Körperfunktionen erlauben. Stellvertretend seien hierfür im Folgenden die SPECT (Single Photon Emission Computer Tomography) und PET (Positron Emission Tomography) betrachtet. Diese Verfahren liefern dreidimensionale Darstellungen der Stoffwechselaktivität des Herzmuskels, die jedoch eine verhältnismäßig schlechte räumliche Auflösung haben, was in vielen Fällen die genaue Diagnose der Herzfunktion erschwert.

Auf der anderen Seite ist es bekannt, dass mit Ultraschallverfahren Schnittbilder des Herzens mit guter Auflösung erzeugt werden können. Beim Ultraschallverfahren im sogenannten B-Modus wird dabei in das Gewebe eine Schallwelle mit ausgeprägter Richtcharakteristik eingestrahlt. Das aus dem Gewebe reflektierte Echosignal wird demoduliert, logarithmiert und gespeichert. Anschließend wird eine neue Schallwelle auf einer anderen Trajektorie abgestrahlt, die aber ebenso wie alle vorhedgsn Schallwellentrajektorien in der Ebene, die abgebildet werden soll, liegt. Nachdem die Ebene auf diese Art befriedigend dicht abgetastet worden ist, werden die demodulierten und logarithmierten Echos als Grauwerte kodiert und in der Bildebene so angeordnet, dass sich eine längen- und winkeltreue Abbildung der Objekte in der abgetasteten Ebene ergibt. Die beschriebene Abtastung der Ebene kann durch eine fächerförmige, parallele oder andersartige Anordnung der Schallwellentrajektorien erreicht werden. Eine Ultraschallaufnahme eines Volumens kann erreicht werden, indem die Trajektorien der Schallwellen so gewählt werden, dass sie nicht in einer Ebene liegen, sondern ein Volumen (z.B. Kegelförmig oder durch Parallelen) abtasten. Den Ultraschallaufnahmen gemeinsam ist, dass sie nur die Anatomie und nicht die Funktion der Herzmuskulatur wiedergeben.

Ferner ist aus der Literatur (J.P. Caravel et al., Fusion d'images anatomique (échographie) et fonctionnelles (tomoscintigraphie) rénales, MÉDECINE NUCLÉAIRE, 1995, Elesvier) ein Verfahren zur Darstellung der Nieren eines Patienten bekannt, wobei von den Nieren zunächst eine dreidimensionale szintigrafische Aufnahme und anschließend eine zweidimensionale Ultraschallaufnahme gemacht wird. Das Ultraschallgerät wird dabei mit Positionsmarkern (Leuchtdioden) versehen, welche von einem Kamerasystem erfasst und mit einem ortsfesten Bezugssystem in Beziehung gebracht werden. Durch entsprechende Berechnungen wird dann die räumliche Lage des Ultraschallgerätes bzw. der Ultraschallaufnahme sowie die Position des szintigrafisch erfassten Körpervolumens ermittelt, woraufhin anschließend aus der dreidimensionalen szintigrafischen Aufnahme ein der Ultraschallaufnahme entsprechender Schnitt berechnet und dem Ultraschallbild überlagert werden kann. Nachteilig bei einem derartigen Verfahren ist jedoch der hohe Aufwand für die optische Positionserfassung von Ultraschallgerät und Szintigraf. Ferner ist die Genauigkeit des Verfahrens durch die Präzision der optischen Positionsbestimmung begrenzt. Ein systematischer Fehlereinfluss liegt weiterhin darin, dass zwischen den Aufnahmen eine Bewegung des Patienten, zum Beispiel durch die Atmung, erfolgen kann, welche dazu führt, dass mit fehlerhaften Positionsannahmen gearbeitet wird.

Aus der Patenschrift US 5,871,013 ist ein Verfahren bekannt, bei dem ein SPTCT-Bild ("Single Photon Transmission Computerized Tomography") oder ein SPECT-Bild mit einem diagnostischen Bild, das ein Ultraschallbild sein kann, registriert. Dabei werden markante anatomische Strukturen in den Bilder verglichen, und mittels Korrelation wird eine Transformation bestimmt, die die Bilder bestmöglich zueinander registriert.

Vor diesem Hintergrund war es eine Aufgabe der vorliegenden Erfindung eine Vorrichtung und ein Verfahren zur Kombination einer Ultraschallaufnahme mit einer nuklearmedizinischen Aufnahme bereitzustellen, welche eine größere Genauigkeit der Registrierung aufweist und mit geringerem Aufwand an spezieller Hardware gewonnen werden kann.

Diese Aufgabe wird durch ein Verfahren mit den Merkmalen des Anspruchs 1 sowie durch eine Vorrichtung mit den Merkmalen des Anspruchs 9 gelöst. Vorteilhafte Ausgestaltungen sind in den Unteransprüchen enthalten.

Das erfindungsgemäße Verfahren zur Verbesserung der Auflösung bzw. Genauigkeit einer nuklearmedizinischen Aufnahme beruht auf der Kombination einer Ultraschallaufnahme aus nuklearmedizinischen Aufnahme beruht auf der Kombination einer Ultraschallaufnahme aus einem Körpervolumen mit der nuklearmedizinisch gewonnenen Aufnahme (im Folgenden oft kurz "Nuklearaufnahme") aus demselben Körpervolumen. Bei dem Verfahren werden verschiedene angenommene räumliche Relativlagen zwischen der Nuklearaufnahme und der Ultraschallaufnahme untersucht, um diejenige Relativlage zu finden, welche der Realität (am besten) entspricht. Für jede angenommene Relativlage wird dabei ein Nuklearteilbild, welches eine echte oder unechte Teilmenge der Nuklearaufnahme ist, mit einem Ultraschallteilbild, welches eine echte oder unechte Teilmenge der Ultraschallaufnahme ist, verglichen, wobei das Nuklearteilbild und das Ultraschallteilbild für die angenommene Relativlage denselben räumlichen Ausschnitt des Körpervolumens abbilden sollen. Der Vergleich erfolgt quantitativ durch die Berechnung eines Bewertungsindexes für die Übereinstimmung zwischen Nuklearteilbild und Ultraschallteilbild. Nach Abschluss der Berechnungen wird diejenige Relativlage als (reale) räumliche Deckungslage von Nuklearteilbild und Ultraschallteilbild ausgewählt, welche den optimalen Wert des Bewertungsindexes aufweist. Je nach Definition des Bewertungsindexes kann eine hohe Übereinstimmung durch einen besonders hohen oder einen besonders niedrigen Wert repräsentiert werden, so dass dementsprechend unter dem optimalen Wert des Bewertungsindexes das Maximum beziehungsweise Minimum aller erhaltenen Werte zu verstehen ist. Im Folgenden soll der üblichen Konvention gefolgt werden, dass ein hoher positiver Wert des Bewertungsindexes eine hohe Übereinstimmung anzeigt, wobei sich alle Ausführungen jedoch analog auch auf alternative Definitionen übertragen lassen.

Eine berorzugte Ausgestaltung des Verfahrens betrifft den Fall, dass die Ultraschallaufnahme zweidimensional und mit den jeweils betrachteten Ultraschallteilbildern identisch ist, und dass die Nuklearteilbilder als zweidimensionale Nuklearschnittbilder aus einer dreidimensionalen nuklearmedizinischen Aufnahme gewonnen werden. Der Vergleich einer zweidimensionalen Ultraschallaufnahme mit einem zweidimensionalen Schnittbild aus einer dreidimensionalen Nuklearaufnahme entspricht einer in der Praxis häufig vorkommenden und daher besonders relevanten Situation. Diese wird daher im Folgenden oft beispielhaft zugrundegelegt.

Das erfindungsgemäße Verfahren umfasst jedoch gleichermaßen einen Vergleich von z.B. dreidimensionalen Ultraschallaufnahmen mit zwei- oder dreidimensionalen Nuklearaufnahmen. Der Vergleich von jeweils dreidimensionalen Aufnahmen kann dabei über Nuklearteilbilder bzw. Ultraschallteilbilder erfolgen, die zweidimensionale Schnitte der dreidimensionalen Aufnahmen darstellen. Insbesondece können Teilbilder bestehend aus drei zueinander orthogonalen Schnitten betrachtet werden.

Das geschilderte Verfahren hat den Vorteil, dass keine aufwendige Apparatur zur Feststellung der relativen räumlichen Lage von Ultraschallaufnahme und Nuklearaufnahme benötigt wird. Vielmehr wird diese mit Hilfe des Bewertungsindexes anhand der bereits vorhandenen Daten ermittelt. So wird z.B. in einer dreidimensionalen nuklearmedizinischen Aufnahme des Körpervolumens dasjenige Nuklearschnittbild gesucht, welches am besten zu einer vorgegebenen zweidimensionalen Ultraschallaufnahme passt, wobei der Arzt die Schnittebene der Ultraschallaufnahme völlig frei wählen kann. Ein weiterer Vorteil des Verfahrens besteht darin, dass es gegenüber einer Bewegung des Patienten zwischen der nuklearmedizinischen Aufnahme und der Ultraschallaufnahme weitgehend robust ist, da diese Bewegung nicht zu einer fehlerhaften Annahme hinsichtlich der Relativlage führen kann.

Bei dem genannten Verfahren wird die nuklearmedizinische Aufnahme des Körpervolumens vorzugsweise mittels einer SPECT oder einer PET gewonnen. Diese Verfahren können dem Arzt wertvolle Informationen z.B. über die Stoffwechselfunktion des Herzmuskels geben, sie sind jedoch beide mit dem Nachteil einer verhältnismäßig geringen räumlichen Auflösung behaftet. Dieser Nachteil kann mit Hilfe des vorgeschlagenen Kombinationsverfahrens wirkungsvoll gemindert werden.

Gemäß einer bevorzugten Ausgestaltung des Verfahrens wird ein Schätzwert für die Lage der Ultraschallaufnahme relativ zum Körpervolumen vorgegeben, und es werden für die Berechnung des Bewertungsindexes dann nur solche Relativlagen zwischen nuklearmedizinischer Aufnahme und Ultraschallaufnahme betrachtet, bei denen die nuklearmedizinische Aufnahme eine angenommene Lage in einer Umgebung dieses Schätzwertes hat. Das heißt zB. für den Vergleich einer dreidimensionalen Nuklearaufnahme mit einer zweidimensionalen Ultraschallaufnahme, dass die Winkel, welche die Lage der Nuklearteilbilder (Nuklearschnittbilder) definieren, aus einem mehr oder weniger großen Intervall um die entsprechenden Winkel-Schätzwerte herum ausgewählt werden. Auf diese Weise kann der Rechenaufwand reduziert und die Gefahr von fehlerhaften Ergebnissen verringert werden. Der Schätzwert für die Lage der Ultraschallaufnahme kann vom aufnehmenden Arzt manuell vorgegeben werden, wobei dieser sich gängiger Klassifizierungen der Aufnahme bedienen kann (beim Herzen zum Beispiel "parasternale Ansicht entlang der Längsachse", "Ansicht der Aortenklappe entlang der kurzen Achse", "apikale Zweikammeransicht", "linke parasternale Ansicht entlang der Längsachse"). Ebenso ist es denkbar, dass automatische Verfahren zur Bestimmung des Schätzwertes verwendet werden, welche nicht unbedingt eine hohe Genauigkeit besitzen müssen.

Weiterhin wird vorzugsweise aus der Ultraschallaufnahme ein charakteristischer Bereich ausgewählt, wobei die anschließende Berechnung der Bewertungsindizes jeweils nur in diesem ausgewählten Bereich stattfindet. Alle anderen Bildregionen spielen dann für die Beurteilung der Übereinstimmung zwischen Ultraschallteilbildern und Nuklearteilbildern keine Rolle. Auf diese Weise kann verhindert werden, dass irrelevante oder verfälschte Bildbereiche auf die Registrierung zwischen Ultraschallteilbild und Nuklearteilbild Einfluss haben. Insbesondere können aus der Ultraschallaufnahme Bereiche mit einer starken Abschattung des Echos oder mit Artefakten ausgeschlossen werden. Ein weiterer Vorteil der Beschränkung auf einen charakteristischen Bereich liegt in der Reduzierung des Rechenaufwandes für die Bestimmung der Bewertungsindizes.

Gemäß einer anderen Weiterbildung des Verfahrens werden Bewertungsindizes auch für solche Bildkombinationen berechnet, bei denen das Ultraschallteilbild relativ zum Nuklearteilbild in mindestens einer Dimension gestreckt oder gestaucht ist. Insbesondere können Versionen der Ultraschallaufnahme verwendet werden, die in Axialrichtung der Aufnahme gesehen und ±10% gestreckt/gestaucht sind, um auf diese Weise Fehler durch eine Zugrundelegung falscher Annahmen für die Ultraschallgeschwindigkeit zu korrigieren.

Um den erforderlichen Rechenaufwand für die Bestimmung der Bewertungsindizes zu minimieren, kann vor der Berechnung die digitale Auflösung der Ultraschallaufnahme und/oder der Nuklearaufnahme reduziert werden. Ferner werden vorteilhafterweise die digitalen Auflösungen beider Bildarten einander angeglichen.

Gemäß einer bevorzugten Ausführungsform des Verfahrens wird der Bewertungsindex zwischen den Ultraschallteilbildern und den Nuklearteilbildern als Summe über alle korrespondierenden Bildpunkte (das heißt Bildpunkte des Ultraschallteilbildes und des Nuklearteilbildes, die als demselben räumlichen Punkt zugehörig angenommen werden) berechnet, wobei jeder Summand

- einen hohen positiven Wert erhält, wenn die verglichenen Bildpunkte des Ultraschallteilbildes und des Nuklearteilbildes beide eine relevante Struktur darstellen;

- einen kleinen Wert erhält, wenn nur der Bildpunkt des Ultraschallteilbildes eine relevante Struktur darstellt;

- einen hohen negativen Wert erhält, wenn nur der Bildpunkt des Nuklearteilbildes eine relevante Struktur darstellt.

Das erfindungsgemäße Verfahren kann insbesondere dadurch weiter gekennzeichnet sein, dass

a) die Definition der Bildpunktwerte der Ultraschallaufnahme so erfolgt, dass Bildpunkte, die zu relevanten Strukturen gehören, große Bildpunktwerte aufweisen. Dies ist bei der üblichen Grauwertdarstellung von Ultraschallaufnahmen der Fall, bei der Herzmuskelgewebe hell, das heißt mit großen Grauwerten dargestellt wird.

b) die Definition der Bildpunktwerte der Nuklearaufnahme so erfolgt, dass Bildpunkte, die zu den in a) genannten relevanten Strukturen gehören, große Bildpunktwerte aufweisen, wenn die Struktur sich in dem spezifischen nuklearmedizinischen Abbildungsverfahren darstellt, und kleine Bildpunktwerte, wenn die Struktur sich nicht darstellt. Eine solche Definition ist bei der SPECT oder PET üblich, wo eine radioaktive Strahlungsaktivität hell, das heißt durch große Grauwerte dargestellt wird.

c) der Bewertungsindex zwischen einem Ultraschallteilbild und einem Nuklearteilbild als Summe über alle korrespondierenden Bildpunkte berechnet wird, wobei jeder in der Summe auftretende und zu zwei korrespondierenden Bildpunkten gehörende Summand

- einen hohen positiven Wert erhält, wenn die verglichenen Bildpunkte des Ultraschallteilbildes und des Nuklearteilbildes beide große Bildpunktwerte aufweisen;

- einen kleinen (positiven oder negativen, nahe bei Null liegenden) Wert erhält, wenn der Bildpunkt des Ultraschallteilbildes einen großen und der entsprechende Bildpunkt des Nuklearteilbildes einen kleinen Bildpunktwert aufweist;

- einen hohen negativen Wert erhält, wenn der Bildpunkt des Ultraschallteilbildes einen kleinen und der entsprechende Bildpunkt des Nuklearteilbildes einen großen Bildpunktwert aufweist.

Ein derartiges Verfahren kann mit den üblichen Darstellungsformen einer Ultraschallaufnahme und einer Nuklearaufnahme ausgeführt werden, bei denen durch ein Ultraschallecho beziehungsweise durch radioaktive Strahlung erfasste Strukturen mit hohen Grauwerten (Bildpunktwerten) abgebildet werden. Es versteht sich jedoch, dass ein äquivalentes Arbeiten auch mit anderen Darstellungsformen möglich wäre. Das Verfahren beruht auf der Annahme, dass sich relevante (anatomische) Strukturen in der Ultraschallaufnahme auf jeden Fall darstellen und daher hier entsprechend hohe Bildpunktwerte haben. In der Nuklearaufnahme stellen sich diese anatomisch vorhandenen Strukturen dagegen nur dann dar, wenn sie eine nuklearmedizinisch nachweisbare Aktivität aufweisen. Hohe Bildpunktwerte im Nuklearteilbild lassen daher auf jeden Fall auf das Vorhandensein einer zugrundeliegenden Struktur schließen, während niedrige Bildpunktwerte (dunkle Bereiche) sowohl durch das Nicht-Vorhandensein von Strukturen als auch durch physiologische Inaktivität vorhandener Strukturen erzeugt sein können. Dieses Verständnis wird bei der oben genannten Definition des Bewertungandexes dadurch berücksichtigt, dass eine zwischen dem Ultraschallteilbild und dem Nuklearteilbild übereinstimmende Detektion von relevanten Strukturen mit einem hohen positiven Wert bewertet wird, dass der Nachweis einer Struktur nur im Ultraschallteilbild mit einem kleinen Wert neutral bewertet wird, und dass der Nachweis einer Struktur nur im Nuklearteilbild mit einem hohen negativen Wert "bestraft" wird, da er im Widerspruch zur Annahme einer räumlichen Deckung der betrachteten Bilder steht.

Gemäß einer Weiterbildung des zuletzt genannten Verfahrens werden die dort genannten hohen positiven beziehungsweise hohen negativen Werte der Summanden betragsmäßig um so größer angesetzt, je sicherer die beteiligten Bildpunktwerte auf die relevante Struktur hinweisen, d.h. - bei der üblichen Definition der Bildpunktwerte - je größer die zugehörigen Bildpunktwerte sind. Hierdurch wird erreicht, dass sicher erkannte Strukturen möglichst stark zum berechneten Bewertungsindex beitragen.

Das geschilderte Verfahren kann insbesondere dazu verwendet werden, das ausgewählte Nuklearteilbild in Kombination mit dem Ultraschallteilbild darzustellen. Dabei kann das Nuklearteilbild zum Beispiel farblich dem Ultraschallteilbild überlagert werden.

Vorzugsweise findet eine solche überlagerte Darstellung nach Art einer Maskierung nur an den Stellen statt, an denen die Bildpunktwerte des Ultraschallteilbildes größer als ein vorgegebener Wert sind Bei einer Untersuchung des Herzens würde das Nuklearteilbild somit nur an den Stellen überlagert, an denen das Ultraschallteilbild Herzmuskelgewebe zeigt.

Die Erfindung betrifft ferner eine Vorrichtung zur (zwei- oder dreidimensionalen) Darstellung eines Körpervolumens, enthaltend

- einen Speicher für eine Ultraschallaufnahme aus dem Körpervolumen;

- einen Speicher für eine nuklearmedizinische Aufnahme aus dem Körpervolumen;

- eine Datenverarbeitungseinheit, welche dazu eingerichtet ist, für verschiedene angenommene Relativlagen zwischen nuklearmedizinischer Aufnahme und Ultraschallaufnahme jeweils für ein Nuklearteilbild und ein Ultraschallteilbild von demselben Ausschnitt des Körpervolumens einen Bewertungsindex betreffend ihre Übereinstimmung zu berechnen und diejenige Relativlage als räumliche Deckungslage von Nuklearteilbild und Ultraschallteilbild auszuwählen, welche den optimalen Wert des Bewertungsindexes aufweist.

Mit einer derartigen Vorrichtung lässt sich das oben erläuterte erfindungsgemäße Verfahren ausführen und die damit mögliche Verbesserung der Genauigkeit nuklearmedizinischer Aufnahmen erzielen. Die Vorrichtung kann insbesondere durch eine entsprechende Programmierung der Datenverarbeitungseinheit so eingerichtet werden, dass sie die Durchführung der verschiedenen beschriebenen Weiterbildungen des Verfahrens erlaubt. Ferner kann die Vorrichtung eine unmittelbare Kopplung mit einem Ultraschallaufnahmegerät und/oder mit einem Tomographen für eine SPECT oder PET aufweisen, um von diesen direkt Bildaufnahmen zu übernehmen.

Im Folgenden wird die Erfindung mit Hilfe der Figuren beispielhaft erläutert. Es zeigt:

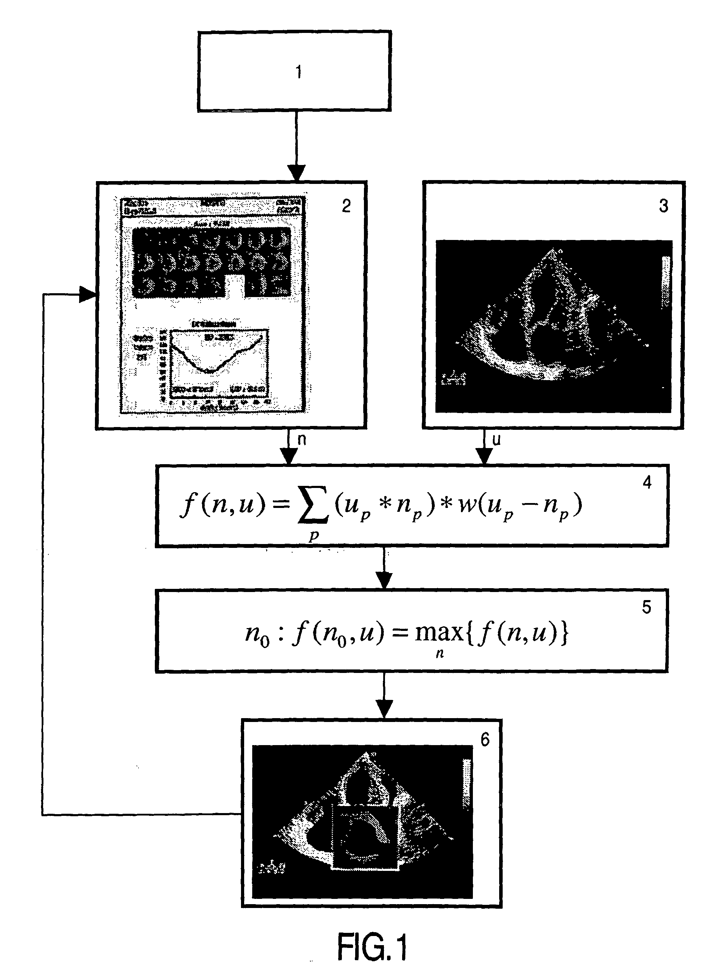

Fig 1     schematisch den Ablauf eines erfindungsgemäßen Verfahrens zur Kombination einer zweidimensionalen Ultraschallaufnahme mit einem zweidimensionalen Schnittbild aus einer nuklearmedizinischen dreidimensionalen Aufnahme;

Fig. 2    das Prinzip einer bildpunktweisen Berechnung eines Bewertungsindexes für die Übereinstimmung zwischen einem Ultraschallteilbild und einem Nuklearteilbild;

Fig. 3    den prinzipiellen Verlauf einer für die Berechnung des Bewertungsindexes verwendeten Funktion.

Mit dem erfindungsgemäßen Verfahren soll eine Ultraschallaufnahme des Herzens, welche eine hohe Ortsauflösung bietet, mit einer nuklearmedizinischen Aufnahme kombiniert werden, welche bei geringer räumlicher Auflösung die Darstellung physiologischer Funktionen ermöglicht. Dabei wird im nachfolgenden Beispiel davon ausgegangen, dass die Ultraschallaufnahme zweidimensional und die nuklearmedizinische Aufnahme dreidimensional sei.

Für die gewünschte Kombination wird zunächst ein nuklearmedizinisches Standardverfahren wie SPECT oder PET eingesetzt, um die in Block 2 angedeutete räumliche Darstellung eines das Herz enthaltenden Körpervolumens zu gewinnen. Nach der Erzeugung dieser dreidimensionalen Aufnahme setzt der Arzt ein herkömmliches, frei bewegliches Ultraschallaufnahmegerät ohne weitere Spezialhardware ein, um ein zweidimensionales Ultraschallbild u des Herzens anzufertigen (Block 3). Der Arzt kann ferner von Hand die ungefähre Lage dieses Ultraschallbildes relativ zum Herzen eingeben (Block 1).

Nach der Bereitstellung des Ultraschallbildes u, der dreidimensionalen nuklearmedizinischen Aufnahme (Block 2), und des Anfangs- oder Schätzwertes für die Lage des Ultraschallbildes (Block 1) kann damit begonnen werden, den zur Ultraschallaufnahme u bestmöglich passenden Schnitt $n_0$ aus der nuklearmedizinischen Volumenaumahme zu bestimmen. Zu diesem Zweck werden in Block 2 zweidimensionale Schnittbilder n durch das dreidimensionale Aufnahmevolumen berechnet. Dabei werden im Voraus einige Schnittbilder aus einem Volumen um den Schätzwert für die Lage des Ultraschallbildes berechnet. Das heißt, dass eine bestimmte Bandbreite an möglichen Schnittlagen um die geschätzte Lage des Ultraschallbildes u herum betrachtet und mit dem Ultraschallbild u verglichen werden soll.

Um dann das am besten zum Ultraschallbild u (das dem "Ultraschallteilbild" des allgemeinen Teils der Beschreibung entspricht) passende Schnittbild $n_0$ zu

bestimmen, werden nachfolgend mit dem Ultraschallbild u und jedem vergleichsweise zugrundegelegten Nuklearschnittbild n (das dem "Nuklearteilbild" des allgemeinen Teils der Beschreibung entspricht) die folgenden Schritte ausgeführt:

1. Das Nuklearschnittbild n wird in seiner Auflösung reduziert, um den Rechenaufwand für die Bildregistrierung zu verringern.

2. Im Ultraschallbild u wird automatisch oder von Hand ein interessierender Bereich ausgewählt, um Regionen mit einer starken Abschattung des Echos oder mit Artefakten auszuschließen.

3. Die Auflösung des Ultraschallbildes u wird so reduziert, dass sie mit derjenigen des Nuklearschnittbildes n übereinstimmt.

4. Der in Block 4 ausgeführte Vergleich des Schnittbildes n und der Ultraschallaufnahme u beruht auf den Annahmen, dass

- das Ultraschallbild u den Herzmuskel mit höheren Grauwerten der Pixel zeigt als das umgebende Gewebe und insbesondere das Lumen des Herzens;

- das Nuklearschnittbild n einen Teil des Herzmuskels zeigt, wobei jedoch auch Teile des Muskels existieren können, die nicht im Bild dargestellt sind.

Unter Berücksichtigung dieser Erwägungen wird im Registrierungsalgorithmus ein Bewertungsindex f(n,u) berechnet, welcher Bereiche positiv bewertet, in denen sowohl im Ultraschallbild u als auch im Nuklearschnittbild n Gewebe sichtbar ist, Bereiche neutral bewertet, in denen nur im Ultraschallbild u Gewebe sichtbar ist, und Bereiche negativ wertet, in denen nur im Nuklearschnittbild n Gewebe sichtbar ist. Der berechnete Bewertungsindex ist ferner lokal mit der Amplitude der Bildsignale der beteiligten Bilder gewichtet. Dabei sei vorausgesetzt, dass die Ultraschallaufnahme u und die Nuklearschnittbilder n hinsichtlich ihres Mittelwertes und der Varianz geeignet vorbehandelt sind.

5. Der wie beschrieben in Block 4 berechnete Bewertungsindex wird in Block 5 mit dem maximalen erhaltenen Bewertungsindex aus den vorherigen Berechnungen verglichen. Ist er größer als dieser, wird sein Wert als maximal erhaltener Bewertungsindex abgespeichert, und das zugrunde liegende Nuklearschnittbild n weist die momentan beste Übereinstimmung mit dem Ultraschallbild u auf. Sodann geht das Verfahren zu Block 2 zurück, wo das nächste Nuklearschnittbild n für den nächsten Vergleich ausgewählt wird, welches eine etwas andere Lage im räumlichen Aufnahmevolumen einnimmt als die vorherigen Schnittbilder.

Um eine fehlerhafte Registrierung aufgrund einer falschen Annahme für die Ultraschallgeschwindigkeit zu vermeiden, kann das Ultraschallbild u vor der Berechnung des Bewertungsindexes in Block 4 in axialer Richtung um bis zu 10% gestreckt werden.

Nachdem alle in Frage kommenden Nuklearschnittbilder n mit dem (gestreckten) Ultraschallbild u verglichen worden sind, steht aus Block 5 das Schnittbild $n_0$ mit der besten Übereinstimmung fest. Dieses wird dann in Block 6 einer Darstellung des Ultraschallbildes überlagert, so dass der behandelnde Arzt sowohl die räumliche Information mit hoher Auflösung aus dem Ultraschallbild als auch die physiologische Information aus dem nuklearmedizinischen Bild zur Verfügung hat.

Bei der gemeinsamen Darstellung kann das nuklearmedizinische Bild n als farbliches overlay zum Ultraschallbild dargestellt werden. Vorzugsweise wird es dabei im Sinne einer Maskierung nur an den Stellen dargestellt, an denen das Ultraschallbild Herzgewebe zeigt. Selbstverständlich sind auch verschiedene Kompromisse zwischen diesen beiden Darstellungsarten möglich.

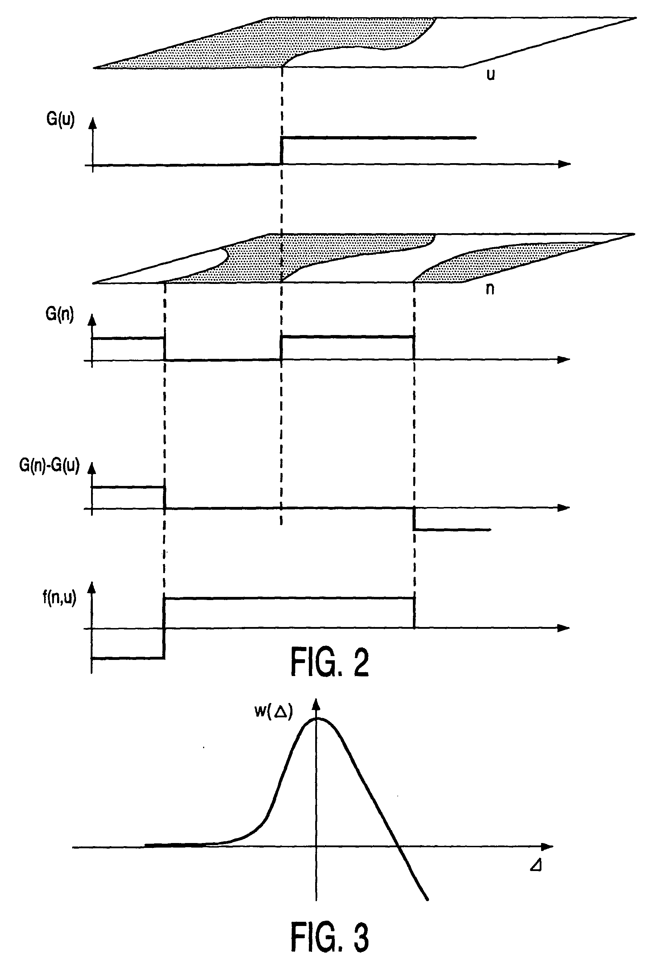

Eine mögliche Definition des in Block 4 verwendeten Bewertungsindexes f(n,u) wird im Folgenden mit Hilfe der Figuren 2 und 3 erläutert.

In Figur 2 sind dabei untereinander schematisch kleine Ausschnitte aus einem zu registrierenden Ultraschallbild u und Nuklearschnittbild n dargestellt. Zur Vereinfachung wird eine binäre Schwarz-Weiß-Darstellung der Bilder angenommen. Das interessierende Herzmuskelgewebe stellt sich im Ultraschallbild u hell dar, was einem großen Grauwert G(u) entspricht (vertikale Achse der zweidimensionalen Diagramme). Ebenso stellt sich im Nuklearschnittbild n eine beobachtete Radioaktivität, die auf eine physiologische Aktivität im Gewebe zurückgeht, hell beziehungsweise durch große Grauwerte G(n) dar.

Im zweituntersten Diagramm von Figur 2 ist in einem eindimensionalen Schnitt die Differenz der Grauwerte zwischen dem Nuklearschnittbild n und dem Ultraschallbild u, G(n) - G(u), dargestellt. Darin sind

- Bereiche positiv, in denen nur das Nuklearschnittbild n Gewebe darstellt,

- Bereiche gleich Null, in denen das Nuklearschnittbild n und die Ultraschallaufnahme u übereinstimmend das Vorhandensein oder Fehlen von Gewebe anzeigen, und

- Bereiche negativ, in denen nur das Ultraschallbild u Gewebe anzeigt.

Würden das Nuklearschnittbild n und das Ultraschallbild u räumlich exakt dieselbe Lage haben, so müsste überall dort, wo das Nuklearschnittbild n Gewebe zeigt, auch das Ultraschallbild u Gewebe zeigen, und dort, wo das Ultraschallbild u kein Gewebe zeigt, dürfte auch das Nuklearschnittbild kein Gewebe zeigen. Bereiche, die diesen Vorstellungen entsprechen, sollen daher im Bewertungsindex hoch beziehungsweise positiv bewertet werden. Bereiche, in denen nur das Nuklearschnittbild Gewebe zeigt, sollen entsprechend negativ bewertet werden. Bereiche, in denen nur das Ultraschallbild u Gewebe zeigt, lassen die Möglichkeit einer räumlichen Übereinstimmung offen und sollen daher neutral bewertet werden. Eine Funktion f(n,u), welche wie im untersten Diagramm von Figur 2 gezeigt diese Kriterien erfüllt, wird z. B. durch

$$f(n,u) = \sum_{p} (u_p \cdot n_p) \cdot w(u_p - n_p)$$

definiert, wobei

p = Bildpunkt

$u_p$ = Bildpunktwert des Bildpunktes p der Ultraschallaufnahme u

$n_p$ = Bildpunktwert des Bildpunktes p des Nuklearschnittbildes n.

Die Funktion w($\Delta$) ist eine Gewichtungsfunktion, welche große negative Differenzen von n und u neutral, kleine Differenzen positiv und große positive Differenzen negativ bewertet. Sie kann zum Beispiel definiert sein als

$$w(\Delta) = w(\Delta'+e) = a \cdot [1+b \cdot \sigma(c\Delta') \cdot (1-d\Delta' \cdot h(\Delta'))],$$

wobei $\Delta' = (\Delta - e)$ ist, $\sigma$ ($\Delta'$) eine sigmoide Funktion ist (stetig, monoton steigend, durch den Ursprung des Koordinatensystems verlaufend und mit Grenzwerten -1 in negativer und +1 in positiver Richtung), und $h(\Delta')$ die Heavysidefunktion (0 für x < 0 und 1 für x > 0) ist, und wobei die reellen Zahlen a, b, c, d, e > 0 Abstimmungsparameter sind. Der Parameter e ist vorzugsweise so gewählt, dass die Funktion w($\Delta$) ihr Maximum bei $\Delta = 0$ hat. Der prinzipielle Verlauf der Gewichtungsfunktion w ist in Figur 3 skizziert.

**Patentansprüche**

1. Verfahren zur Verbesserung der Auflösung einer nuklearmedizinischen Aufnahme aus einem Körpervolumen durch ihre Kombination mit einer Ultraschallaufnahme aus demselben Körpervolumen, wobei für verschiedene angenommene Relativlagen zwischen nuklearmedizinischer Aufnahme und Ultraschallaufnahme jeweils für ein Nuklearteilbild und ein Ultraschallteilbild von demselben Ausschnitt des Körpervolumens ein Bewertungsindex betreffend ihre Übereinstimmung berechnet wird, und wobei diejenige Relativlage als räumliche Deckungslage von Nuklearteilbild und Ultraschallteilbild ausgewählt wird, welche den optimalen Wert des Bewertungsindexes aufweist, **dadurch gekennzeichnet,** **dass** der Bewertungsindex zwischen dem Ultraschallteilbild und dem Nuklearteilbild als Summe über alle korrespondierenden Bildpunkte berechnet wird, wobei jeder Summand

   - einen hohen positiven Wert erhält, wenn die verglichenen Bildpunkte beide eine relevante Struktur darstellen;
   - einen kleinen Wert erhält, wenn nur der Bildpunkt des Ultraschallteilbildes eine relevante Struktur darstellt;
   - einen hohen negativen Wert erhält, wenn nur der Bildpunkt des Nuklearteilbildes eine relevante Struktur darstellt.

2. Verfahren nach Anspruch 1, **dadurch gekennzeichnet,** **dass** die Ultraschallaufnahme zweidimensional und mit den jeweils betrachteten Ultraschallteilbildern identisch ist, und dass die Nuklearteilbilder als zweidimensionale Nuklearschnittbilder aus einer dreidimensionalen nuklearmedizinischen Aufnahme gewonnen werden.

3. Verfahren nach Anspruch 1, **dadurch gekennzeichnet,** **dass** die nuklearmedizinische Aufnahme des Körpervolumens mit einer SPECT oder einer PET gewonnen wird.

4. Verfahren nach Anspruch 1, **dadurch gekennzeichnet,** **dass** ein Schätzwert für die Lage der Ultraschallaufnahme relativ zum Körpervolumen vorgegeben wird, und dass für die Berechnung des Bewertungsindexes nur Relativlagen zwischen nuklearmedizinischer Aufnahme und Ultraschallaufnahme betrachtet werden, bei denen die nuklearmedizinische Aufnahme eine Lage in einer Umgebung dieses Schätzwertes hat.

5. Verfahren nach Anspruch 1, **dadurch gekennzeichnet,** **dass** aus der Ultraschallaufnahme ein charakteristischer Bereich ausgewählt wird, und dass der Bewertungsindex nur für diesen Bereich berechnet wird.

6. Verfahren nach Anspruch 1,
   **dadurch gekennzeichnet,**
   **dass** die Berechnung des Bewertungsindexes auch für Nuklearteilbilder und Ultraschallteilbilder erfolgt, die relativ zueinander in mindestens einer Dimension gestreckt und/oder gestaucht sind.

7. Verfahren nach Anspruch 1,
   **dadurch gekennzeichnet,**
   **dass** die hohen positiven beziehungsweise hohen negativen Werte betragsmäßig um so größer sind, je sicherer die beteiligten Bildpunktwerte auf die relevante Struktur hinweisen.

8. Verfahren nach Anspruch 1,
   **dadurch gekennzeichnet,**
   **dass** Bildpunkte des Nuklearteilbildes dort den Bildpunkten des als räumlich deckungsgleich ermittelten Ultraschallteilbildes überlagert dargestellt werden, wo die entsprechenden Bildpunktwerte des Ultraschallteilbildes größer als ein vorgegebener Grenzwert sind.

9. Vorrichtung zur Darstellung eines Körpervolumens, enthaltend

   - einen Speicher für eine Ultraschallaufnahme aus dem Körpervolumen;
   - einen Speicher für eine nuklearmedizinische Aufnahme aus dem Körpervolumen;
   - eine Datenverarbeitungseinheit, welche dazu eingerichtet ist, für verschiedene angenommene Relativlagen zwischen nuklearmedizinischer Aufnahme und Ultraschallaufnahme jeweils für ein Nuklearteilbild und ein Ultraschallteilbild von demselben Ausschnitt des Körpervolumens einen Bewertungsindex betreffend ihre Übereinstimmung zu berechnen und diejenige Relativlage als räumliche Deckungslage von Nuklearteilbild und Ultraschallteilbild auszuwählen, welche den optimalen Wert des Bewertungsindexes aufweist,

   **dadurch gekennzeichnet, dass** die Datenverarbeitungseinheit weiterhin dazu eingerichtet ist, den Bewertungsindex zwischen dem Ultraschallteilbild und dem Nuklearteilbild als Summe über alle korrespondierenden Bildpunkte zu berechnen, wobei jeder Summand

   - einen hohen positiven Wert erhält, wenn die verglichenen Bildpunkte beide eine relevante Struktur darstellen;
   - einen kleinen Wert erhält, wenn nur der Bildpunkt des Ultraschallteilbildes eine relevante Struktur darstellt;

   - einen hohen negativen Wert erhält, wenn nur der Bildpunkt des Nuklearteilbildes eine relevante Struktur darstellt.

## Claims

1. A method of improving the resolution of a nuclear medical image of a body volume by combining it with an ultrasound image of the same body volume, in which for various assumed relative positions of the nuclear medical image and the ultrasound image each time an evaluation index concerning the correspondence of a nuclear sub-image and an ultrasound sub-image of the same region of the body volume is calculated, and the relative position having the optimum value of the evaluation index is selected as the spatial registration position of the nuclear sub-image and the ultrasound sub-image, **characterized in that** the evaluation index between the ultrasound sub-image and the nuclear sub-image is calculated as the sum over all corresponding image points, each summand

   - being assigned a large positive value if the compared image points both represent a relevant structure;
   - being assigned a small value if only the image point of the ultrasound sub-image represents a relevant structure;
   - being assigned a large negative value if only the image point of the nuclear sub-image represents a relevant structure.

2. A method as claimed in claim 1, **characterized in that** the ultrasound image is a two-dimensional image which is identical to the respective ultrasound sub-images considered, and that the nuclear sub-images are derived as two-dimensional nuclear sectional images from a three-dimensional nuclear medical image.

3. A method as claimed in claim 1, **characterized in that** the nuclear image of the body volume is acquired by means of a SPECT or a PET method.

4. A method as claimed in claim 1, **characterized in that** an estimated value is provided for the position of the ultrasound image relative to the body volume and that the calculation of the evaluation index takes into account only those relative positions of the nuclear image and the ultrasound image where the nuclear medical image occupies a position in a vicinity of this estimated value.

5. A method as claimed in claim 1, **characterized in that** a characteristic region is selected from the ultrasound image and that the evaluation index is calculated only for this region.

6. A method as claimed in claim 1, **characterized in that** the calculation of the evaluation index is performed also for nuclear sub-images and ultrasound sub-images which have been stretched and/or compressed relative to one another in at least one dimension.

7. A method as claimed in claim 1, **characterized in that** the absolute values of the large positive or high negative values are larger as the image point values involved point more reliably towards the relevant structure.

8. A method as claimed in claim 1, **characterized in that** wherever the corresponding image point values of the ultrasound sub-image are larger than a predetermined limit value, image points of the nuclear sub-image are reproduced in superposed form on the image points of the spatially registering ultrasound sub-image.

9. A device for the imaging of a body volume, which device includes

   - a memory for an ultrasound image of the body volume;
   - a memory for a nuclear medical image of the body volume;
   - a data processing unit which is arranged to calculate, for various assumed positions of the nuclear medical image and the ultrasound image, an evaluation index concerning the correspondence of each time a nuclear sub-image and an ultrasound sub-image of the same region of the body volume and to select the relative position which has the optimum value of the evaluation index as the spatial registration position of the nuclear sub-image and the ultrasound sub-image, **characterized in that** the data processing unit is further arranged to calculate the evaluation index between the ultrasound sub-image and the nuclear sub-image as the sum over all corresponding image points, each summand
   - being assigned a large positive value if the compared image points both represent a relevant structure;
   - being assigned a small value if only the image point of the ultrasound sub-image represents a relevant structure;
   - being assigned a large negative value if only the image point of the nuclear sub-image represents a relevant structure.

## Revendications

1. Procédé pour améliorer la résolution d'une image de médecine nucléaire à partir d'un volume corporel par sa combinaison avec une image aux ultrasons à partir du même volume corporel,
   dans lequel, pour différentes positions relatives adoptées entre l'image de médecine nucléaire et l'image aux ultrasons ou pour une image partielle nucléaire et une image partielle aux ultrasons du même segment du volume corporel, un indice d'évaluation concernant leur correspondance est calculé et
   dans lequel la position relative est sélectionnée comme une position de couverture spatiale de l'image partielle nucléaire et de l'image partielle aux ultrasons qui présente la valeur optimale de l'indice d'évaluation,
   **caractérisé en ce**
   **que** l'indice d'évaluation entre l'image partielle aux ultrasons et l'image partielle nucléaire est calculé comme la somme de tous les points d'image correspondants, dans lequel chaque terme de la somme:

   - reçoit une valeur positive élevée lorsque les points d'image comparés représentent tous deux une structure pertinente;
   - reçoit une valeur minime lorsque seul le point d'image de l'image partielle aux ultrasons représente une structure pertinente;
   - reçoit une valeur négative élevée lorsque seul le point d'image de l'image partielle nucléaire représente une structure pertinente.

2. Procédé selon la revendication 1,
   **caractérisé en ce**
   **que** l'image aux ultrasons est bidimensionnelle et identique aux images partielles aux ultrasons respectivement considérées et que les images partielles nucléaires sont obtenues comme des coupes nucléaires bidimensionnelles à partir d'une image tridimensionnelle de médecine nucléaire.

3. Procédé selon la revendication 1,
   **caractérisé en ce**
   **que** l'image de médecine nucléaire du volume corporel est obtenue avec une SPECT ou une PET.

4. Procédé selon la revendication 1,
   **caractérisé en ce**
   **qu'**une estimation est préalablement déterminée pour la position de l'image aux ultrasons par rapport au volume corporel et que, pour le calcul de l'indice d'évaluation, seules des positions relatives sont prises en considération entre l'image de médecine nucléaire et l'image aux ultrasons, l'image de médecine nucléaire ayant une position dans un environnement de cette estimation.

5. Procédé selon la revendication 1, **caractérisé en ce** qu'une zone caractéristique est sélectionnée à partir de l'image aux ultrasons et que l'indice d'évaluation n'est calculé que pour cette zone.

6. Procédé selon la revendication 1, **caractérisé en ce** que le calcul de l'indice d'évaluation est effectué également pour les images partielles nucléaires et pour les images partielles aux ultrasons qui sont étirées et/ou aplaties l'une par rapport à l'autre dans au moins une dimension.

7. Procédé selon la revendication 1, **caractérisé en ce** que les valeurs positives élevées ou négatives élevées sont d'autant plus grandes sur le plan chiffré que les valeurs de point d'image impliquées renvoient avec plus de certitude à la structure pertinente.

8. Procédé selon la revendication 1, **caractérisé en ce** que les points d'image de l'image partielle nucléaire y sont représentés superposés aux points d'image de l'image partielle aux ultrasons déterminée comme de couverture équivalente spatialement, où les valeurs de point d'image correspondantes de l'image partielle aux ultrasons sont supérieures à une valeur-limite préalablement déterminée.

9. Dispositif de représentation d'un volume corporel contenant

   - une mémoire pour une image aux ultrasons du volume corporel;
   - une mémoire pour une image de médecine nucléaire du volume corporel;
   - une unité de traitement de données qui est organisée afin de calculer, pour différentes positions relatives adoptées entre une image de médecine nucléaire et une image aux ultrasons ou pour une image partielle nucléaire et une image partielle aux ultrasons du même fragment du volume corporel, un indice d'évaluation concernant leur correspondance et afin de sélectionner la position relative comme position de couverture de l'image partielle nucléaire et de l'image partielle aux ultrasons qui présente la valeur optimale de l'indice d'évaluation, **caractérisé en ce** que l'unité de traitement de données est par ailleurs organisée pour calculer l'indice d'évaluation entre l'image partielle aux ultrasons et l'image partielle nucléaire comme somme de tous les points d'image correspondants, dans lequel chaque terme de la somme

   - reçoit une valeur positive élevée lorsque les points d'image comparés représentent tous deux une structure pertinente;
   - reçoit une valeur minime lorsque seul point d'image de l'image partielle aux ultrasons représente une structure pertinente;
   - reçoit une valeur négative élevée lorsque seul le point d'image de l'image partielle nucléaire représente une structure pertinente.

$$f(n,u) = \sum_{p} (u_p * n_p) * w(u_p - n_p)$$

$$n_0 : f(n_0, u) = \max_{n} \{ f(n, u) \}$$

FIG.1

FIG. 2

FIG. 3